(19) Europäisches Patentamt
European Patent Office
Office européen des brevets

(11) Veröffentlichungsnummer: **0 144 740**
**B1**

# (12) EUROPÄISCHE PATENTSCHRIFT

(45) Veröffentlichungstag der Patentschrift: **11.10.89**

(51) Int. Cl.[4]: **A 61 K 7/13**

(21) Anmeldenummer: **84113214.5**

(22) Anmeldetag: **02.11.84**

(54) **Haarfärbemittel.**

(30) Priorität: **11.11.83 DE 3340829**

(43) Veröffentlichungstag der Anmeldung: **19.06.85 Patentblatt 85/25**

(45) Bekanntmachung des Hinweises auf die Patenterteilung: **11.10.89 Patentblatt 89/41**

(84) Benannte Vertragsstaaten: **AT BE CH DE FR GB IT LI LU NL SE**

(56) Entgegenhaltungen:
**EP-A- 0 039 807**
**DE-A- 3 233 541**
**DE-A- 3 330 623**
**US-A- 3 006 963**
**US-A- 4 207 110**

(73) Patentinhaber: **Henkel Kommanditgesellschaft auf Aktien**
**Postfach 1100 Henkelstrasse 67**
**D-4000 Düsseldorf-Holthausen (DE)**

(72) Erfinder: **Maak, Norbert, Dr.**
**Liebigstrasse 18**
**D-4040 Neuss (DE)**
Erfinder: **Lieske, Edgar**
**Hunsrückenstrasse 40**
**D-4000 Düsseldorf (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 144 740 B1

**Beschreibung**

Gegenstand der Erfindung sind Haarfärbemittel auf der Basis von Oxidationsfarbstoffen. Solche Haarfärbemittel enthalten Oxidationsfarbstoffvorprodukte in einem kosmetischen Träger. Als Oxidationsfarbstoffvorprodukte werden Entwicklersubstanzen und Kupplersubstanzen eingesetzt, die unter dem Einfluß von Oxidationsmitteln oder von Luftsauerstoff Farbstoffe ausbilden. Als kosmetische Träger für die Oxidationsfarbstoffvorprodukte dienen Cremes, Emulsionen, Gele, Shampoos, Schaumaerosole oder andere Zubereitungen, die für die Anwendung auf dem Haar geeignet sind.

Für das Färben von Haaren spielen die sogenannten Oxidationsfarben, die durch oxidative Kupplung einer oder mehrerer Entwicklerkomponenten untereinander oder mit einer oder mehreren Kupplerkomponenten entstehen, wegen ihrer intensiven Farben und guten Echtheitseigenschaften eine bevorzugte Rolle. Als Entwicklersubstanzen werden üblicherweise primäre aromatische Amine mit einer weiteren in Para- oder Orthoposition befindlichen freien oder substituierten Hydroxy- oder Aminogruppe, ferner Diaminopyridinderivate, heterocyclische Hydrazonderivate, 4-Aminopyrazolonderivate und Tetraaminopyrimidine eingesetzt. Als sogenannte Kupplersubstanzen werden m-Phenylendiaminderivate, Naphthole, Resorcinderivate und Pyrazolone verwendet. Gute Oxidationshaarfarbstoffvorprodukte müssen in erster Linie folgende Voraussetzungen erfüllen. Sie müssen bei der oxidativen Kupplung die gewünschten Farbnuancen in ausreichender Intensität ausbilden. Sie müssen ferner ein gutes Aufziehvermögen auf menschliches Haar besitzen ohne die Kopfhaut zu stark anzufärben und sie sollen vor allem in toxikologischer und dermatologischer Hinsicht unbedenklich sein.

Die Verwendung von Resorcin als Kuppler ist aus DE-C-162 625 und DE-C-276 761 bekannt. Darüber hinaus sind weitere Resorcinderivate als Kuppler für Oxidationshaarfärbemittel vorgeschlagen worden, zum Beispiel Mono- und Dialkyl-m-dihydroxybenzole in der DE-C-26 17 739. Resorcin und die bekannten Resorcinderivate befriedigen jedoch nicht in den Echtheitseigenschaften der damit erzielbaren Haarfärbungen. Aus der älteren DE-A1-33 30 623 ist die Verwendung von 2,2',4,4'-Tetrahydroxy-diphenylsulfid als Wirkstoff gegen Kopfschuppen und Komponente von Oxidationshaarfärbemitteln beschrieben. Im Hinblick auf diese Patentanmeldung werden gesonderte Patentansprüche für die Bundesrepublik Deutschland aufgestellt.

Es wurde nun gefunden, daß Haarfärbemittel, enthaltend Oxidationsfarbstoffvorprodukte in einem kosmetischen Träger, die als Oxidationsfarbstoffvorprodukte Verbindungen der allgemeinen Formel

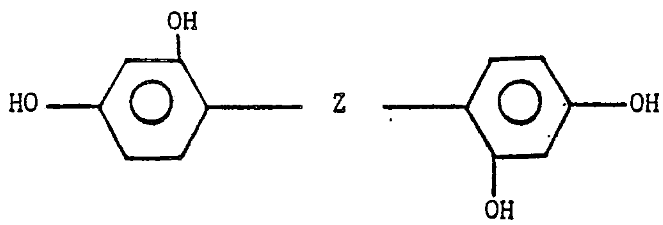

in der Z ein Schwefelatom, eine Gruppe SO oder eine Gruppe $SO_2$ bedeutet, oder deren Salze als Kupplerkomponente und die in Oxidationsfärbemitteln üblichen Entwicklersubstanzen enthalten, die gestellten Anforderungen in hohem Maße erfüllen und darüber hinaus ganz überraschend den Vorteil einer geringeren Hautanfärbung aufweisen.

Die erfindungsgemäßen Haarfärbemittel liefern besonders intensive und brillante Haarfärbungen von hoher Lichtechtheit und Wärmestabilität, deren Nuancen vornehmlich im Braunbereich liegen.

Die erfindungsgemäß einzusetzenden Kupplerverbindungen der allgemeinen Formel sind die literaturbekannten Verbindungen Diresorcylsulfid, Diresorcylsulfoxid und Diresorcylsulfon. Die Herstellung von Diresorcylsulfid ist bekannt aus US-A-2.760.989, die Herstellung des Diresorcylsulfoxids aus US-A-3.006.963 und die Herstellung des Diresorcylsulfons aus DE-C-1 112 282.

Als Entwickler können alle hierfür bekannten Verbindungen eingesetzt werden. Besonders wertvolle Farbnuancen werden erhalten, wenn als Entwicklersubstanzen aromatische und/oder heterocyclische Diamine enthalten sind. Solche Entwicklersubstanzen sind zum Beispiel p-Phenylendiamin, p-Toluylendiamin, N-Methyl-p-phenylendiamin, N,N-Dimethyl-p-phenylendiamin, N,N-Diethyl-2-methyl-p-phenylendiamin, N-Ethyl-N-(2-hydroxyethyl)-p-phenylendiamin, Chlor-p-phenylendiamin, N,N-Bis(2-hydroxyethyl)-p-phenylendiamin, Methoxy-p-phenylendiamin, 2.6-Dichlor-p-phenylendiamin, 2-Chlor-6-brom-p-phenylendiamin, 2-Chlor-6-methyl-p-phenylendiamin, 6-Methoxy-3-methyl-p-phenylendiamin, 2.5-Diaminoanisol, N-(2-Hydroxypropyl)-p-phenylendiamin, N-2-Methoxyethyl-p-phenylendiamin und andere Verbindungen der genannten Art, die weiterhin eine oder mehrere $NH_2$-Gruppen, NHR-Gruppen, $NR_2$-Gruppen, wobei R einen Alkylrest mit 1-4 oder einen Hydroxyalkylrest mit 2-4 Kohlenstoffatomen darstellt, ferner Diaminopyridinderivate, N-Butyl-N-sulfobutyl-p-phenylendiamin sowie besonders Tetraaminopyrimidine wie 2.4.5.6-Tetraaminopyrimidin, 4,5-Diamino-2,6-bismethylaminopyrimidin, 2,5-Diamino-4-diethylamino-6-methylaminopyrimidin, 2.4.5-Triamino-6-dimethylaminopyrimidin, 2.4.5-Triamino-6-piperidinopyrimidin, 2.4.5-Triamino-6-anilino-pyrimidin, 2.4.5-Triamino-6-morpholinopyrimidin, 2.4.5-Triamino-6-(2-hydroxyethyl)-aminopyrimidin.

Mit 2.4.5.6-Tetraaminopyrimidin und/oder dessen Derivaten als Entwicklersubstanz werden besonders wertvolle, rotbraune Nuancen erhalten.

Die erfindungsgemäßen Haarfärbemittel können zusätzlich übliche Kupplersubstanzen, wie zum Beispiel m-Phenylendiaminderivate, Phenole, Naphthole oder Pyrazolone enthalten. Auch direktziehende Farbstoffe, zum Beispiel Nitrophenylendiaminderivate, können zur Modifikation der Farbnuancen zugesetzt werden.

In den erfindungsgemäßen Haarfärbemitteln werden die Kuppler der allgemeinen Formel und die gegebenenfalls zusätzlich vorhandenen anderen Kupplersubstanzen im allgemeinen in etwa molaren Mengen, bezogen auf die verwendeten Entwicklersubstanzen eingesetzt. Wenn sich auch der molare Einsatz als zweckmäßig erweist, so ist es jedoch nicht nachteilig, einen gewissen Überschuß einzelner Oxidationsfarbstoffvorprodukte zum Einsatz zu bringen.

Es ist auch nicht erforderlich, daß die Kuppler der allgemeinen Formel sowie die sonst in den Haarfärbemitteln vorhandenen Oxidationsfarbstoffvorprodukte oder direkt ziehenden Farbstoffe einheitliche chemische Verbindungen darstellen. Vielmehr können diese auch Gemisch der erfindungsgemäß einzusetzenden Kuppler- oder Entwicklersubstanzen sein.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt am Haar gewünscht wird. Als Oxidationsmittel kommen insbesondere Wasserstoffperoxid oder dessen Anlagerungsprodukte an Harnstoff, Melamin oder Natriumborat sowie Gemische aus derartigen Wasserstoffperoxidanlagerungsprodukten mit Kaliumperoxiddisulfat in Betracht.

Zur Herstellung der erfindungsgemäßen Haarfärbemittel werden die Oxidationsfarbstoffvorprodukte in einen geeigneten kosmetischen Träger eingearbeitet. Solche Träger sind zum Beispiel Cremes, Emulsionen, Gele oder auch tensidhaltige, schäumende Lösungen, zum Beispiel Shampoos oder andere Zubereitungen, die für die Anwendung auf dem Haar geeignet sind. Übliche Bestandteile solcher kosmetischer Zubereitungen sind zum Beispiel Netz- und Emulgiermittel, wie anionische, nichtionische oder ampholytische Tenside, zum Beispiel Fettalkoholsulfate, Alkansulfonate, α-Olefinsulfonate, Fettalkoholpolyglycolethersulfate, Ethylenoxidanlagerungsprodukte an Fettalkohole, Fettsäuren und Alkylphenole, Sorbitanfettsäureester und Fettsäurepartialglyceride, Fettsäurealkanolamide sowie Verdickungsmittel, wie zum Beispiel Methyl- oder Hydroxymethylcellulose, Stärke, Fettalkohole, Paraffinöle, Fettsäuren, ferner Parfümöle und haarpflegende Zusätze, wie zum Beispiel wasserlösliche kationische Polymere, Proteinderivate, Pantothensäure oder Cholesterin. Die Bestandteile der kosmetischen Träger werden zur Herstellung der erfindungsgemäßen Haarfärbemittel in für diese Zwecke üblichen Mengen eingesetzt; zum Beispiel werden Emulgiermittel in Konzentrationen von 0,5 bis 30 Gewichtsprozent und Verdickungsmittel in Konzentrationen von 0,1 bis 25 Gewichtsprozent des gesamten Färbemittels eingesetzt. Die Oxidationsfarbstoffvorprodukte werden in Mengen von 0,2 bis 5 Gewichtsprozent, vorzugsweise 1 bis 3 Gewichtsprozent des gesamten Färbemittels in den Träger eingemischt.

Die Anwendung der erfindungsgemäßen Haarfärbemittel kann, unabhängig von der Art der kosmetischen Zubereitung, zum Beispiel als Creme, Gel oder Shampoo, im schwach sauren, neutralen oder alkalischen Milieu erfolgen. Bevorzugt ist die Anwendung der Haarfärbemittel in einem pH-Bereich von 8-10. Die Anwendungstemperaturen können in einem Bereich zwischen 15 und 40 °C liegen. Nach einer Einwirkungszeit von ca. 30 Minuten wird das Haarfärbemittel durch Ausspülen von dem zu färbenden Haar entfernt. Danach wird das Haar mit einem milden Shampoo nachgewaschen und getrocknet. Das Nachwaschen mit einem Shampoo entfällt, wenn ein stark tensidhaltiger Träger, zum Beispiel ein Färbeshampoo, verwendet wurde.

Die mit den erfindungsgemäßen Haarfärbemitteln erzielbaren Färbungen haben eine hohe Brillanz sowie überlegene Wärme-, Licht-, Wasch- und Reibechtheitseigenschaften. Die nachfolgenden Beispiele sollen den Erfindungsgegenstand näher erläutern, ohne ihn jedoch hierauf zu beschränken.

## Beispiele

Es wurden erfindungsgemäße Haarfärbemittel in Form einer Haarfärbe-Cremeemulsion der folgenden Zusammensetzung hergestellt.

| | |
|---|---|
| Fettalkohol $C_{12-18}$ | 10 g |
| Fettalkohol $C_{12-14}$ + 2 EO-sulfat, Na-Salz, 28 %ig | 25 g |
| Wasser | 60 g |
| Kupplersubstanz | 0,0075 Mol |
| Entwicklersubstanz | 0,0075 Mol |
| $Na_2SO_3$ (Inhibitor) | 1,0 g |
| konzentrierte Ammoniak-Lösung | bis pH = 9,5 |
| Wasser | ad 100 g |

Die Bestandteile wurden der Reihe nach miteinander vermischt. Nach Zugabe der Oxidationsfärbemittelvorprodukte und der Inhibitors wurde zunächst mit konzentrierter Ammoniak-Lösung der pH-Wert der Emulsion auf 9,5 eingestellt, dann wurde mit Wasser auf 100 g aufgefüllt.

Die oxidative Entwicklung der Färbung wurde mit 3 %iger Wasserstoffperoxidlösung als Oxidationsmittel durchgeführt. Hierzu wurden 100 g der Emulsion mit 50 g Wasserstoffperoxidlösung (3 %ig) versetzt und vermischt.

Die Färbecreme wurde auf ca. 5 cm lange Strähnen standardisierten, zu 90 % ergrauten, aber nicht besonders vorbehandelten Menschenhaars aufgetragen und dort 30 Minuten bei 27 °C belassen. Nach Beendigung des Färbeprozesses wurde das Haar gespült, mit einem üblichen Haarwaschmittel ausgewaschen und anschließend getrocknet.

Als Kupplersubstanzen wurden die folgenden Verbindungen eingesetzt:

K 1: Diresorcylsulfid

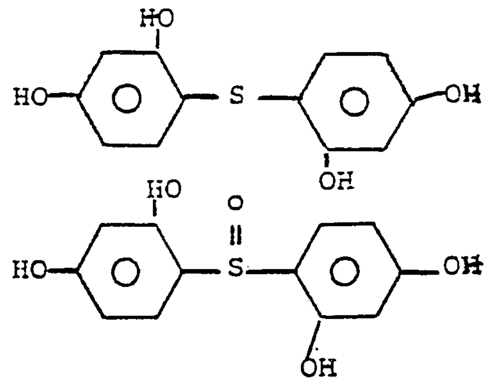

K 2: Diresorcylsulfoxid

Als Entwicklersubstanzen wurden die folgenden Verbindungen eingesetzt:

E 1 : 2.4.5.6-Tetraaminopyrimidin
E 2 : p-Phenylendiamin
E 3 : p-Toluylendiamin
E 4 : 2.5-Diaminoanisol
E 5 : 2-Chlor-o-phenylendiamin
E 6 : N-Ethyl-N-hydroxyethyl-p-phenylendiamin
E 7 : N,N-Dimethyl-p-phenylendiamin
E 8 : p-Aminophenol

Die mit diesen Oxidationsfarbstoffvorprodukten erhaltenen Färbungen sind der Tabelle I zu entnehmen.

Tabelle I

| Beispiel | Entwickler | Kuppler | erhaltener Farbton |
|---|---|---|---|
| 1 | E 1 | K 1 | rotbraun |
| 2 | E 2 | K 1 | dunkelbraun |
| 3 | E 3 | K 1 | dunkelbraun |
| 4 | E 4 | K 1 | dunkelbraun |
| 5 | E 5 | K 1 | olivbraun |
| 6 | E 6 | K 1 | dunkelbraun |
| 7 | E 7 | K 1 | dunkelbraun |
| 8 | E 8 | K 1 | olivbraun |
| 9 | E 1 | K 2 | rotbraun |
| 10 | E 2 | K 2 | dunkelbraun |
| 11 | E 3 | K 2 | dunkelbraun |
| 12 | E 4 | K 2 | dunkelbraun |
| 13 | E 5 | K 2 | olivbraun |
| 14 | E 6 | K 2 | aubergine |
| 15 | E 7 | K 2 | dunkelbraun |
| 16 | E 8 | K 2 | olivbraun |

**Patentansprüche** (für die Vertragsstaaten : AT, BE, CH, FR, GB, IT, LI, LU, NL, SE)

1. Haarfärbemittel, enthaltend Oxidationsfarbstoffvorprodukte in einem kosmetischen Träger, dadurch gekennzeichnet, daß als Oxidationsfarbstoffvorprodukte Verbindungen der allgemeinen Formel

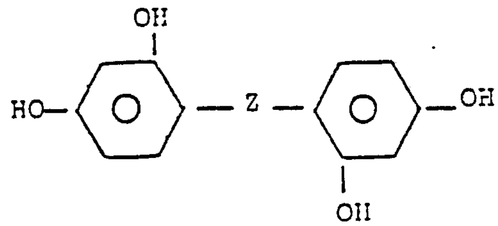

in der Z ein Schwefelatom, eine Gruppe SO oder eine Gruppe $SO_2$ bedeutet, oder deren Salze als Kupplerkomponente und die in Oxidationsfärbemitteln üblichen Entwicklersubstanzen enthalten sind.

2. Haarfärbemittel nach Anspruch 1, dadurch gekennzeichnet, daß als Entwicklersubstanzen aromatische und/oder heterocyclische Diamine enthalten sind.

3. Haarfärbemittel nach Anspruch 1, dadurch gekennzeichnet, daß als Entwicklersubstanzen 2.4.5.6-Tetraaminopyrimidin und/oder Derivate davon enthalten sind.

4. Haarfärbemittel nach Anspruch 1 bis 3, dadurch gekennzeichnet, daß zusätzlich weitere, bekannte Kupplersubstanzen und/oder direkt ziehende Farbstoffe enthalten sind.

5. Haarfärbemittel nach Anspruch 1 bis 4, dadurch gekennzeichnet, daß der Gehalt an Oxidationsfarbstoffvorprodukten 0,2 bis 5,0 Gew.% des gesamten Färbemittels ausmacht.

**Patentansprüche** (für den Vertragsstaat DE)

1. Haarfärbemittel, enthaltend Oxidationsfarbstoffvorprodukte in einem kosmetischen Träger, dadurch gekennzeichnet, daß als Oxidationsfarbstoffvorprodukte Verbindungen der allgemeinen Formel

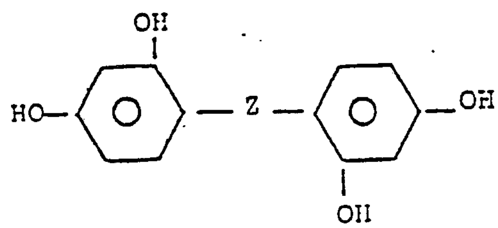

in der Z eine Gruppe SO oder eine Gruppe $SO_2$ bedeutet, oder deren Salze als Kupplerkomponente und die in Oxidationsfärbemitteln üblichen Entwicklersubstanzen enthalten sind.

2. Haarfärbemittel nach Anspruch 1, dadurch gekennzeichnet, daß als Entwicklersubstanzen aromatische und/oder heterocyclische Diamine enthalten sind.

3. Haarfärbemittel nach Anspruch 1, dadurch gekennzeichnet, daß als Entwicklersubstanzen 2.4.5.6-Tetraaminopyrimidin und/oder Derivate davon enthalten sind.

4. Haarfärbemittel nach Anspruch 1 bis 3, dadurch gekennzeichnet, daß zusätzlich weitere, bekannte Kupplersubstanzen und/oder direktziehende Farbstoffe enthalten sind.

5. Haarfärbemittel nach Anspruch 1 bis 4, dadurch gekennzeichnet, daß der Gehalt an Oxidationsfarbstoffvorprodukten 0,2 bis 5,0 Gew.-% des gesamten Färbemittels ausmacht.

**Claims** (for the Contracting States : AT, BE, CH, FR, GB, IT, LI, LU, NL, SE)

1. Hair dyes containing oxidation dye precursors in a cosmetic carrier, characterized in that the oxidation dye precursors present are compounds corresponding to the following general formula

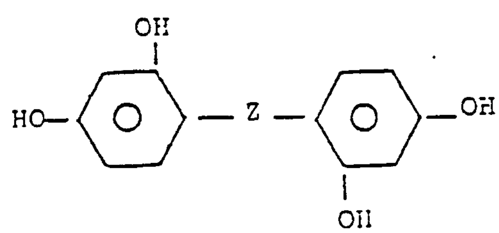

in which Z is a sulfur atom, a group SO or a group $SO_2$, or salts thereof, as coupler component and the developers normally used in oxidation hair dyes.

2. Hair dyes as claimed in Claim 1, characterized in that aromatic and/or heterocyclic diamines are present as the developer substances.

3. Hair dyes as claimed in Claim 1, characterized in that 2,4,5,6-tetraaminopyrimidine and/or derivatives thereof are present as the developer substances.

4. Hair dyes as claimed in Claims 1 to 3, characterized in that other known coupler substances and/or substantive dyes are additionally present.

5. Hair dyes as claimed in Claims 1 to 4, characterized in that the oxidation dye precursors make up from 0.2 to 5.0 % by weight of the dye as a whole.

**Claims** (for the Contracting State DE)

1. Hair dyes containing oxidation dye precursors in a cosmetic carrier, characterized in that the oxidation dye precursors present are compounds corresponding to the following general formula

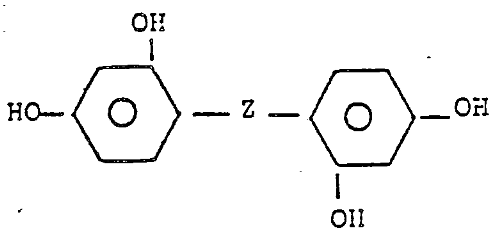

in which Z is a group SO or a group $SO_2$, or salts thereof, as coupler component ant the developers normally used in oxidation hair dyes.

2. Hair dyes as claimed in Claim 1, characterized in that aromatic and/or heterocyclic diamines are present as the developer substances.

3. Hair dyes as claimed in Claim 1, characterized in that 2,4,5,6-tetraaminopyrimidine and/or derivatives thereof are present as the developer substances.

4. Hair dyes as claimed in Claims 1 to 3, characterized in that other known coupler substances and/or substantive dyes are additionally present.

5. Hair dyes as claimed in Claims 1 to 4, characterized in that the oxidation dye precursors make up from 0.2 to 5.0 % by weight of the dye as a whole.

**Revendications** (pour les Etats contractants : AT, BE, CH, FR, GB, IT, LI, LU, NL, SE)

1. Produit de teinture pour cheveux, contenant des précurseurs de colorants d'oxydation dans un véhicule cosmétique, caractérisé en ce qu'il contient, en tant que précurseurs de colorants d'oxydation, des composés de formule générale :

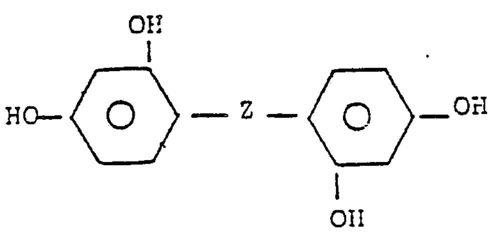

dans laquelle Z représente un atome de soufre, un groupe SO ou un groupe $SO_2$, ou des sels de ceux-ci, et les substances de développement usuelles dans des colorants d'oxydation.

2. Produit de teinture pour cheveux selon la revendication 1, caractérisé en ce que, en tant que substances de développement, il contient des diamines aromatiques et/ou hétérocycliques.

3. Produit de teinture pour cheveux selon la revendication 1, caractérisé en ce que, en tant que substances de développement, il contient de la 2,4,5,6-tétraaminopyrimidine et/ou des dérivés de celle-ci.

4. Produit de teinture pour cheveux selon l'une quelconque des revendications 1 à 3, caractérisé en ce qu'il contient en outre d'autres substances de copulation connues et/ou des colorants directs.

5. Produit de teinture pour cheveux selon l'une quelconque des revendications 1 à 4, caractérisé en ce que la teneur en précurseurs de colorants d'oxydation représente de 0,2 à 5,0 % en poids de la composition totale de teinture.

**Revendications** (pour l'Etat contractant DE)

1. Produit de teinture pour cheveux, contenant des précurseurs de colorants d'oxydation dans un véhicule cosmétique, caractérisé en ce qu'il contient, en tant que précurseurs de colorants d'oxydation, des composés de formule générale :

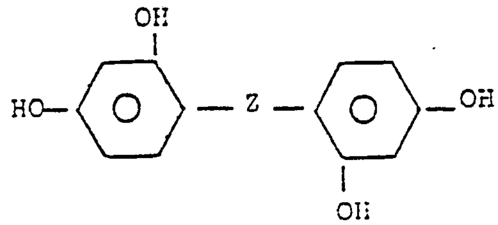

dans laquelle Z représente un groupe SO ou un groupe $SO_2$, ou des sels de ceux-ci, et les substances de développement usuelles dans des colorants d'oxydation.

2. Produit de teinture pour cheveux selon la revendication 1, caractérisé en ce que, en tant que substances de développement, il contient des diamines aromatiques et/ou hétérocycliques.

3. Produit de teinture pour cheveux selon la revendication 1, caractérisé en ce que, en tant que substances de développement, il contient de la 2,4,5,6-tétraaminopyrimidine et/ou des dérivés de celle-ci.

4. Produit de teinture pour cheveux selon l'une quelconque des revendications 1 à 3, caractérisé en ce qu'il contient en outre d'autres substances de copulation connues et/ou des colorants directs.

5. Produit de teinture pour cheveux selon l'une quelconque des revendications 1 à 4, caractérisé en ce que la teneur en précurseurs de colorants d'oxydation représente de 0,2 à 5,0 % en poids de la composition totale de teinture.